# EUROPEAN PATENT APPLICATION

(11) **EP 2 937 036 A1**
(43) Date of publication of application: **28.10.2015**
(21) Application number: 12890272.3
(22) Date of filing: 20.12.2012
(51) Int. Cl.: A61B 5/0408, A61B 5/0478, A61B 5/0492

(54) **BRAIN ELECTRODE SYSTEM**

(71) Applicant: Tohoku-microtec Co., Ltd., Sendai-shi, Miyagi 980-8579 (JP); Tohoku University, Sendai-shi, Miyagi 980-8577 (JP)
(72) Inventor: MOTOYOSHI, Makoto, Sendai-shi Miyagi 980-8579 (JP); KOYANAGI, Mitsumasa, Sendai-shi Miyagi 980-8577 (JP); MUSHIAKE, Hajime, Sendai-shi Miyagi 980-8577 (JP); IWASAKI, Masaki, Sendai-shi Miyagi 980-8577 (JP); KATAYAMA, Norihiro, Sendai-shi Miyagi 980-8577 (JP)
(74) Representative: FDST Patentanwälte
(86) International application number: PCT/JP2012/083090
(87) International publication number: WO 2014/097450

(57) **Abstract**

A brain electrode system comprises: a brain electrode body which is placed in the cranium and has an electrode which detects a brain wave signal and a first coil through which an electric current corresponding to the brain wave signal flows; and a communication unit which is disposed on the scalp, has a second coil which magnetically connects with the first coil and in which an induced electromotive force occurs due to a change in the current that flows through the first coil and receives the brain wave signal with the second coil.

## Description

### TECHNICAL FIELD

The present invention relates to a brain electrode system that detects and measures a brain wave by a brain electrode placed in the cranium.

### BACKGROUND ART

Along with the graying of society in recent years, patients who developed diseases caused by brain disorders (for example, Parkinson's disease, motor paralysis, epilepsy, etc.) have increased. For the development of therapy of diseases caused by such brain disorders, elucidation of brain functions, in particular, elucidation of the activity of neural circuits in the brain is essential. For this purpose, there has been conventionally used a probe-shaped brain electrode (brain probe) that is inserted in the brain and detects electrical signals in the brain cells (a brain wave). Further, in the treatment of diseases caused by brain disorders, in order to identify the part of the brain in which the disorder has occurred, a brain probe is also used as means for providing electrical stimulation to the inside of the brain.

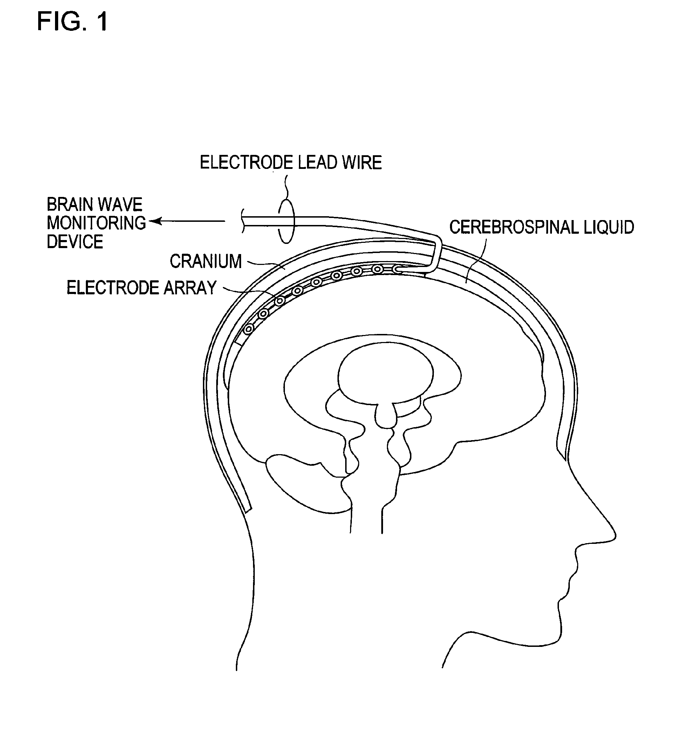

In particular, in the surgical treatment of intractable epilepsy, continuous monitoring of the brain wave for about two weeks is required to find a brain region that is the cause of the seizures. At present, for the measurement of the brain wave to diagnose epilepsy, there has been used an intracranial brain wave (Intracanical EEG) method in which a brain electrode is placed in the cranium to implement monitoring. In the brain wave measurement by the brain electrode system according which the brain electrode is placed in the cranium, in order to identify the seizure origin part, a subdural electrode array in which a plurality of electrodes are arranged in a matrix shape (Patent Literature 1, for example) is disposed on a surface of the brain subdural space, or a deep brain electrode in a thin bar (a probe) shape (Non-patent Literature 1, for example) is inserted deep into the cerebrum. A wire is pulled out from the electrode placed in the cranium, and is connected to an external measurement system. The brain wave is recorded for about two weeks. FIG. 1 is a view illustrating a configuration example of pulling the wire out from the electrode placed in the cranium and connecting the wire to the external measurement system.

### CITATION LIST

### PATENT LITERATURE

Patent Literature 1: Japanese Patent Application Laid-open No. 2009-45368

### NON-PATENT LITERATURE

Non-patent Literature 1: Japanese Journal of Applied Physics 48 (2009) 04C194

### SUMMARY OF INVENTION

### PROBLEMS TO BE SOLVED BY THE INVENTION

To perform electroencephalography, for about two weeks, a patient needs to spend in a state that the wire is pulled out from the brain electrode placed in the cranium, and is forced considerable inconvenience during the monitoring period, so that the burden of the patient is large.

Therefore, an object of the present invention is to provide a brain electrode system that reduces the burden on the patient, in the brain electrode system that measures a brain wave by having a brain electrode placed in the cranium.

### MEANS FOR SOLVING THE PROBLEMS

A brain electrode system of the present invention that achieves the above object includes: a brain electrode body that is placed in a cranium, and that has an electrode which detects a brain wave signal and a first coil through which a current corresponding to the brain wave signal flows; and a communication unit that is disposed on a scalp, has a second coil which is magnetically coupled to the first coil and in which an induced electromotive force occurs based on a change in the current flowing through the first coil, and that receives the brain wave signal with the second coil.

### ADVANTAGEOUS EFFECTS OF THE INVENTION

According to the present invention, the brain wave signal detected by the electrode placed in the cranium can be transferred wirelessly to the communication unit on the scalp. The wire to be pulled out from the electrode placed in the cranium is not required. Inconvenience of the patient during the monitoring period can be relaxed, and the burden of the patient can be substantially reduced.

### BRIEF DESCRIPTION OF DRAWINGS

FIG. 1 is a view illustrating a configuration example of pulling a wire from an electrode placed in a cranium and connecting the wire to an external measurement system.
FIG. 2 is a view illustrating a configuration example of a brain electrode system according to an embodiment of the present invention.
FIG. 3 is a view illustrating a configuration example of a brain electrode body 10.
FIG. 4 is a diagram illustrating a transmission and reception configuration between the brain electrode body 10 and a communication unit 20.
FIG. 5 illustrates dependency data of an induced electromotive force by magnetic coupling on a distance between a primary coil and a secondary coil, deviation between coil centers, and a coil inclination angle (angle deviation).

### DESCRIPTION OF EMBODIMENT

Hereinafter, an embodiment of the present invention will be described with reference to the drawings. However, the embodiment does not limit the technical range of the present invention.

A brain electrode system according to the present embodiment is a system that wirelessly transmits a brain wave signal detected by an electrode placed in cranium and that receives the brain wave signal by a communication unit fitted to the scalp.

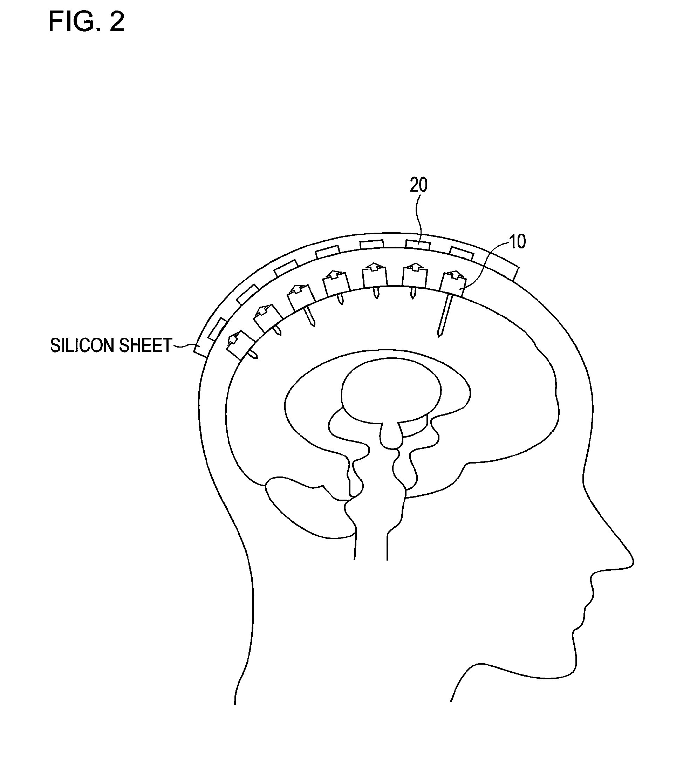

FIG. 2 is a view illustrating a configuration example of the brain electrode system according to the embodiment of the present invention. The brain electrode system is configured by at least one brain electrode body 10 positioned in the cranium, and at least one communication unit 20 disposed on a scalp. A brain wave signal detected by the brain electrode body 10 is wirelessly transmitted by magnetic coupling between a primary coil at a brain electrode body 10 side and a secondary coil at a communication unit 20 side described later, and is received by the communication unit 20.

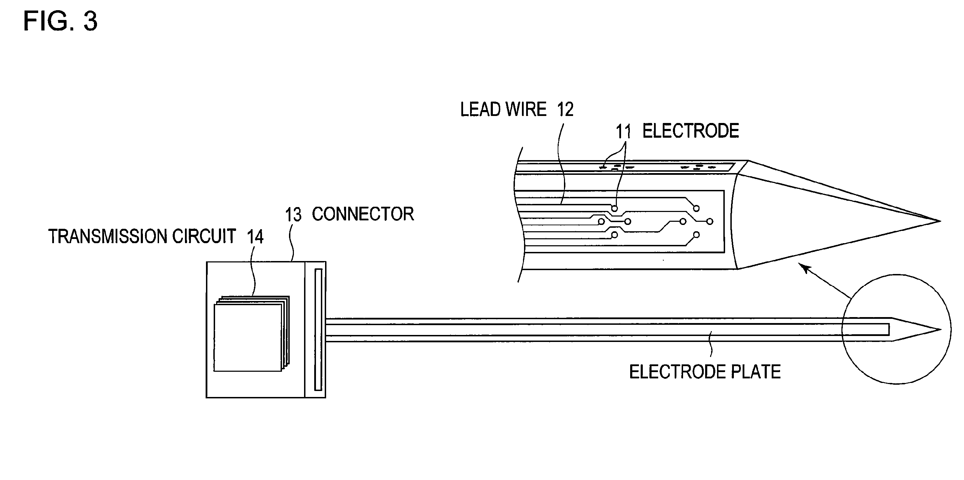

FIG. 3 is a diagram illustrating a configuration example of the brain electrode body 10. Each of the plurality of electrodes 10 placed in the cranium is a brain probe inserted into the brain to detect an electrical signal in the brain (a brain wave signal), and has a configuration of having at least one electrode 11 disposed at a front end of a thin probe made of flexible resin or made of metal (tungsten, for example) and having a diameter of about a few hundred microns, for example. The brain probe illustrated in FIG. 3 has a configuration having an electrode plate of silicon or an electrode plate made of resin (polyimide, for example) fitted to a surface of the probe made of metal. A length of the probe is appropriately selected depending on the measurement point in the brain (the brain surface or the deep cerebral). An optimum shape and an optimum configuration of the probe can be also suitably selected.

The electrode 11 is electrically connected to a connector 13 of a base portion at the other end side of the probe, via a lead wire 12 extended from the electrode 11. The connector 13 is connected with a transmission circuit 14. The transmission circuit 14 is an IC chip that includes an amplifier circuit, a filter circuit, a modulation circuit, a primary coil, and the like, and that has a function of modulating and analog transmitting a brain wave signal detected by the electrode 11. By analog transmitting the brain wave signal, it is possible to maintain the IC chip more compact, and suppress power consumption (heat generation). Certainly, it is also possible to convert the brain wave signal into a digital signal and transmit the digital signal.

Because a distance between the coils, which extends from the cranium through the skull to the scalp, is a few cm, little power is used for the transmission. The brain electrode member 10 placed in the skull is mounted with a micro-cell serving as a power source for the brain electrode body 10. The micro-cell is small and has lightweight sufficient enough to place the micro-cell in the cranium, and has a sufficient capacity as a brain wave measurement power source for about a two-week diagnosis of epilepsy, for example.

Power consumption is determined such that a temperature increase is less than 1°C under the European standards. The periphery of the brain electrode body 10 (the brain probe) is covered with cerebrospinal fluid, and the cerebrospinal fluid of an adult amounts to about 150 ml in total. Because the cerebrospinal fluid is produced by about 500 ml per day and is circulating at the speed of this degree, a thermal diffusion speed is fast. In addition, when the brain probe has been embedded in a tissue of rich blood circulation (such as the surface of the brain, and under the scalp), efficient heat dissipation by blood flow can be expected. Assuming a case where a specific heat of the cerebrospinal fluid is substantially 1 and there is no dissipation of heat, time for increasing by 1°C the temperature of the cerebrospinal fluid of a volume of 1 ml to power consumption of 1 mW of the IC chip in the cranium is 4200 seconds (70 minute). When average power consumption per one brain electrode body is 1 mW, it is possible to suppress with no problem the temperature increase to equal to or lower than 1°C. Regarding power supply, when a monitoring period with power consumption of 1 mW is about a few weeks, a micro-cell can sufficiently supply the power, and non-contact power supply by a magnetic field also becomes possible.

In the case of configuring the brain electrode body 10 by a silicon substrate, in addition to forming on the silicon substrate the electrode 11 and the lead wire 12, by using a manufacturing process of an LSI, an element that configures the transmission circuit 14 can be directly formed on the silicon substrate the device. This enables the transmission function to be incorporated into the brain electrode body 10 itself, and integrally manufacture the transmission circuit and the brain probe.

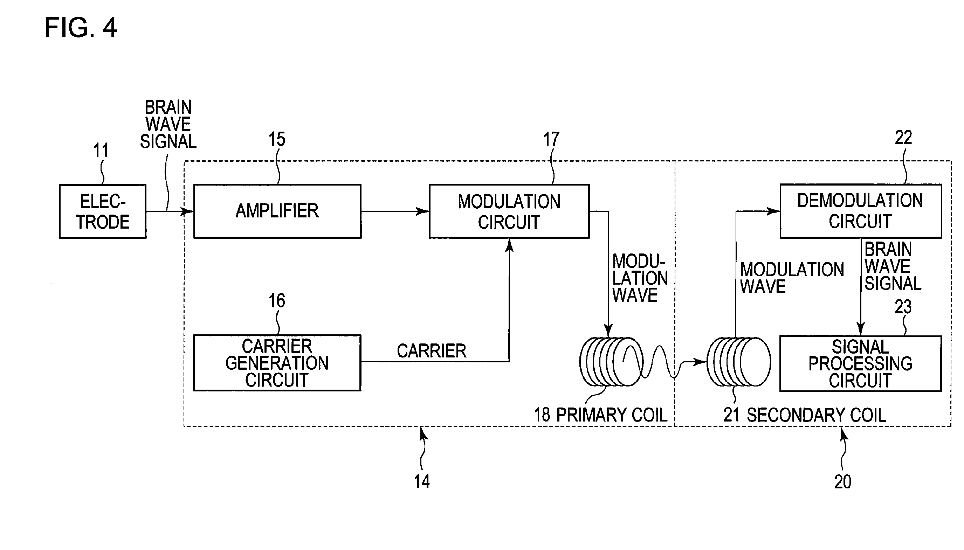

FIG. 4 is a diagram illustrating a transmission and reception configuration between the brain electrode body 10 and the communication unit 20. The brain wave signal detected by the electrode 11 is amplified by an amplifier 15 of the transmission circuit 14, and thereafter, is modulated by a modulation circuit 17. The modulation circuit 17, as an example, modulates the amplitude of the amplified brain wave signal, by using a predetermined carrier wave generated by a carrier generation circuit 16. This modulation signal is supplied to a coil (a primary coil) 18, and a current corresponding to the level of the modulation signal flows through the coil 18.

A coil (a secondary coil) 21 of the communication unit 20 is disposed at a position where the coil 21 is magnetically coupled to the coil 18 of the brain electrode body 10. When an induced electromotive force corresponding to a current change of the coil 18 occurs, the modulation signal from the coil 18 is wirelessly transmitted, and is received by the coil 21 of the communication unit 20. A demodulation circuit 22 of the communication unit 20 demodulates the modulation signal from the coil 21, and returns the demodulated result to the brain wave signal.

The brain wave signal received by the communication unit 20 is transmitted to an external brain wave monitoring device (not illustrated) by wire or wirelessly. A signal processing circuit 23 of the communication unit 20 performs various signal processing operations such as an amplifying processing of the received brain wave signal and an A/D conversion processing, and transmits the brain wave signal to the brain wave monitoring device.

The communication unit 20 is formed as a circuit chip including the coil 21, and is fitted to the surface of a sheet using silicon as a material, for example. By adhering the sheet to the scalp, the communication unit 20 is disposed on the scalp.

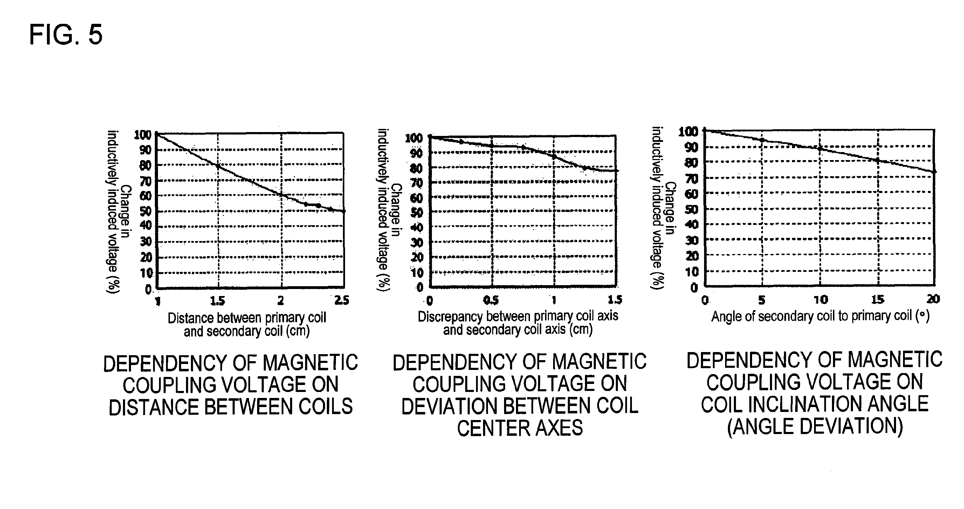

A deviation may arise between a position of the communication unit 20 on the scalp and a position of the brain electrode member 10 in the cranium during operation. FIG. 5 illustrates dependency data of an induced electromotive force by magnetic coupling on a distance between a primary coil and a secondary coil, deviation between coil centers, and a coil inclination angle (angle deviation). As illustrated in FIG. 5, signal intensity decreases depending on the distance and deviation. Preferably, on the silicon sheet, a plurality of communication units 20 are arranged on a matrix at regular intervals. Out of the plurality of communication units 20 arranged on the scalp, a signal received by the communication unit 20 having the best reception sensitivity (strongest magnetic coupling) is used.

As described above, the brain electrode system according to the present embodiment has a configuration in which the brain wave signal detected by the electrode placed in the cranium is wirelessly transmitted to the communication unit that is fitted to the scalp. Accordingly, the wire to be pulled to the outside of the brain from the electrode placed in the cranium is not needed. By mitigating the inconvenience of the patient during the monitoring period, the burden on the patient is greatly reduced.

It is also considered that wireless transmission is directly performed from the brain electrode body placed in the cranium to the brain wave monitoring device that is installed outside without depending on the communication unit disposed on the scalp. However, based on at least the following reasons (1) and (2), in the present embodiment, wireless transmission of the brain wave signal is realized by short-range communication from within the cranium to the scalp, by utilizing magnetic coupling between the primary coil and the secondary, as described above.

(1) Because the brain electrode body 10 is placed in the cranium, a transmission function portion thereof is located in the cerebrospinal fluid of the electrolyte. Therefore, weak electric wave is absorbed. In addition, the output varies greatly depending on the movement of a person (patient). Therefore, it is difficult to transmit the brain wave signal wirelessly to the brain wave monitoring device several meters away from the inside of the cranium.

(2) In order to transmit the electric wave to a distance of a few meters, a large power is needed. Further, in order to suppress noise, conversion to digital data is needed. However, because a filter, an A/D converter, and a transmission amplifier are needed, this leads to the increase in chip size in the cranium and increase in power, and placement thereof in the cranium is difficult.

Further, the brain probe configuring the brain electrode body 10 is used not only to detect and transmit the brain wave signal but also to perform electrical stimulation. Therefore, the transmission circuit 14 of the brain electrode body 10 may have not only a function of detecting and transmitting the brain wave but also a function of receiving a signal for the electrical stimulation from the outside, and transmitting the signal to the electrode. Then, the communication unit 20 has a function of transmitting to the brain electrode body 10 the electrical stimulation signal. Wireless transmission from the communication unit 20 to the brain electrode body 10 is also realized by magnetic coupling between the coil (the primary coil) 21 of the communication unit 20 and the coil (the secondary coil) 18 of the brain electrode body 10. Accordingly, because both the brain electrode body 10 and the communication unit 20 have a function of transmission and reception, bidirectional wireless communications become possible. Further, a brain probe having not only the electrical stimulation function but also a light stimulation function and further a liquid injection function has been put into practical use. By using this brain probe as the brain electrode body 10, and by transmitting an external control signal to the brain probe in the cranium by the wireless communication proposed by the present invention, it becomes possible to realize sophisticated diagnosis and even treatment, by the wireless brain electrode body placed in the cranium.

Accordingly, techniques of the present invention can be applied to areas of movement disorders other than epilepsy (tremor, dystonia), depression, neurodegenerative disorders, headache, migraine, intractable pain, rehabilitation and functional reconstruction.

The present invention is not limited to the above embodiment. Needless to mention, various modifications and design alterations including corrections within a range not deviating from the scope that a person having ordinary knowledge in the art of the present invention can easily reach are all included in the present invention.

### INDUSTRIAL APPLICABILITY

The present invention can be applied to an electrode probe, which is inserted into the brain, of a device that measures a brain wave and a device that provides electrical stimulation to the brain cells.

### REFERENCE SIGNS LIST

10: BRAIN ELECTRODE BODY
11: ELECTRODE
12: LEAD WIRE
13: CONNECTOR
14: TRANSMISSION CIRCUIT
16: CARRIER GENERATION CIRCUIT
17: MODULATION CIRCUIT
18: COIL
20: COMMUNICATION UNIT
21: COIL
22: DEMODULATION CIRCUIT
23: SIGNAL PROCESSING CIRCUIT

## Claims

1. A brain electrode system comprising:
a brain electrode body that is placed in a cranium, and that has an electrode which detects a brain wave signal and a first coil through which a current corresponding to the brain wave signal flows; and
a communication unit that is disposed on a scalp, has a second coil which is magnetically coupled to the first coil and in which an induced electromotive force occurs based on a change in the current flowing through the first coil, and that receives the brain wave signal with the second coil.

2. The brain electrode system according to claim 1, wherein
the communication unit transmits a received brain wave signal to an external brain wave monitoring device that is connected by wire or wirelessly.

3. The brain electrode system according to claim 1, wherein
the brain electrode body is a probe, at a front end of which the electrode is disposed, a circuit including the first coil is formed at a base portion on an opposite end side to the front end, and the circuit is electrically connected to the electrode.

4. The brain electrode system according to claim 1, wherein
a plurality of communication units are fitted to a sheet to be adhered to a scalp.

5. The brain electrode system according to claim 1, wherein
by magnetic coupling between the first coil and the second coil, the brain electrode body and the communication unit perform bidirectional communications.
